# EUROPEAN PATENT APPLICATION

(11) **EP 4 834 366 A1**
(43) Date of publication of application: **04.11.2026**
(21) Application number: 24913725.8
(22) Date of filing: 27.12.2024
(51) Int. Cl.: A61K 39/395, A61K 9/08, A61K 47/26, A61K 47/10, A61K 47/18, A61K 47/20, A61K 47/22, A61K 9/00, A61P 35/00, C07K 16/28

(54) **STABLE PHARMACEUTICAL PREPARATION**

(30) Priority: 29.12.2023 KR 20230196397
(71) Applicant: Celltrion, Inc., Incheon 22014 (KR)
(72) Inventor: SHIN, Yeon-Kyeong, Incheon 22014 (KR); KIM, Kwang-Woo, Incheon 22014 (KR); KIM, Su-Jung, Incheon 22014 (KR); KIM, Su-Ha, Incheon 22014 (KR); OH, Jun-Seok, Incheon 22014 (KR); LEE, Jae-Bin, Incheon 22014 (KR); CHUNG, Sung-Eun, Incheon 22014 (KR); HAN, Won-Yong, Incheon 22014 (KR)
(74) Representative: EIP
(86) International application number: PCT/KR2024/021358
(87) International publication number: WO 2025/143921

(57) **Abstract**

A stable pharmaceutical preparation according to the present invention includes: (A) an antibody or antigen-binding fragment thereof that binds to PD-1 (CD279, programmed cell death protein 1); (B) a surfactant; (C) a stabilizer; (D) a buffer; and (E) an antioxidant. The stable pharmaceutical preparation according to the present invention is stable despite containing the antibody that binds to PD-1 (CD279, programmed cell death protein 1), has excellent long-term storage stability based on excellent stability especially under accelerated conditions and harsh conditions, and may be administered intravenously or subcutaneously.

## Description

### Technical Field

The present invention relates to a stable pharmaceutical formulation including an antibody or an antigen-binding fragment thereof that binds to PD-1 (CD279, programmed cell death protein 1).

### Background Art

PD-1 (CD279, programmed cell death protein 1) is a surface protein of activated T cells, and PD-L1 (CD274, programmed death-ligand 1) and PD-L2 (CD273, programmed cell death-ligand 2), which are surface proteins of cancer cells, can neutralize T cells by binding to PD-1. Cancer immunotherapy drugs can inhibit the evasion function of cancer cells by preventing the binding of PD-L1 and PD-L2 to PD-1, thereby preventing the worsening of symptoms of PD-1-related diseases.

Pembrolizumab, which may be a type of cancer immunotherapy drug, is a humanized monoclonal antibody that selectively binds to and neutralizes PD-1 (CD279, programmed cell death protein 1), and was approved for use by the EMA (European Medicines Agency) and the FDA (U.S. Food and Drug Administration) in July 2015 and October 2016, respectively.

However, antibodies that bind to PD-1 are relatively unstable proteins, and can undergo physical or chemical changes due to heat stress, light stress, etc. Thus, when the products are stored for a long period of time, the stability and/or activity thereof may be impaired.

International Patent Publication No. WO2012-135408 discloses a lyophilized pharmaceutical formulation comprising an anti-human PD-1 antibody. However, such a formulation has the inconvenience of having to be reconstituted with a diluent before use, and the reconstitution step increases the possibility of contamination of the pharmaceutical formulation.

Therefore, there is a need for the development of a stable pharmaceutical formulation that does not require a reconstitution step after lyophilization and has excellent long-term storage stability while containing an antibody.

### [Prior Art Documents]

### [Patent Documents]

International Patent Publication No. WO2012-135408

### DISCLOSURE

### Technical Problem

A problem to be solved by the present invention is to provide a stable pharmaceutical formulation including an antibody that binds to PD-1 (CD279, programmed cell death protein 1).

Another problem to be solved by the present invention is to provide a vial filled with the pharmaceutical formulation.

Still another problem to be solved by the present invention is to provide a cartridge filled with the pharmaceutical formulation.

Yet another problem to be solved by the present invention is to provide a pre-filled syringe filled with the pharmaceutical formulation.

Still yet another problem to be solved by the present invention is to provide an auto-injector including the pre-filled syringe therein.

### Technical Solution

To solve the above problems, the present invention provides a stable pharmaceutical formulation including: (A) an antibody or antigen-binding fragment thereof that binds to PD-1 (CD279, programmed cell death protein 1); (B) a surfactant; (C) a stabilizer; (D) a buffer; and (E) an antioxidant.

In one embodiment of the present invention, the antibody or antigen-binding fragment thereof (A) may include: a light-chain variable region including a CDR1 domain comprising the amino acid sequence of SEQ ID NO:1, a CDR2 domain comprising the amino acid sequence of SEQ ID NO: 2, and a CDR3 domain comprising the amino acid sequence of SEQ ID NO: 3; and a heavy-chain variable region including a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

In one embodiment of the present invention, the antibody or antigen-binding fragment thereof (A) may include a light-chain variable region comprising the amino acid sequence of SEQ ID NO: 7, and a heavy-chain variable region comprising the amino acid sequence of SEQ ID NO: 8.

In one embodiment of the present invention, the antibody or antigen-binding fragment thereof (A) may include a light chain comprising the amino acid sequence of SEQ ID NO: 9, and a heavy chain comprising the amino acid sequence of SEQ ID NO: 10.

In one embodiment of the present invention, the antibody or antigen-binding fragment thereof (A) may be pembrolizumab.

In one embodiment of the present invention, the pharmaceutical formulation may be in a liquid state.

In one embodiment of the present invention, the pharmaceutical formulation may be used for treating diseases associated with PD-1 (CD279, Programmed cell death protein 1).

In one embodiment of the present invention, the concentration of the antibody or antigen-binding fragment thereof (A) may be 1 to 300 mg/ml.

In one embodiment of the present invention, the surfactant (B) may include polysorbate, poloxamer, or a mixture thereof.

In one embodiment of the present invention, the polysorbate may include at least one selected from the group consisting of polysorbate 20, polysorbate 40, polysorbate 60, and polysorbate 80.

In one embodiment of the present invention, the polysorbate may include polysorbate 80.

In one embodiment of the present invention, the concentration of the surfactant (B) may be 0.001 to 1% (w/v).

In one embodiment of the present invention, the stabilizer (C) may include at least one selected from the group consisting of an amino acid or amino acid derivative, and a sugar or sugar alcohol.

In one embodiment of the present invention, the amino acid or amino acid derivative may include at least one selected from the group consisting of threonine, arginine, proline, leucine, glycine, phenylalanine, tryptophan, glutamine, aspartate, glutamate, alanine, asparagine, serine, and tyrosine.

In one embodiment of the present invention, the sugar or sugar alcohol may include at least one selected from the group consisting of trehalose, mannitol, sorbitol, glucose, fructose, galactose, xylose, maltose, lactose, xylitol, D-maltitol, inositol, lactitol, and isomalt.

In one embodiment of the present invention, the concentration of the amino acid or amino acid derivative may be 10 to 450 mM.

In one embodiment of the present invention, the concentration of the sugar or sugar alcohol may be 0.1 to 30% (w/v).

In one embodiment of the present invention, the stabilizer (C) may include sorbitol.

In one embodiment of the present invention, the buffer (D) may include at least one selected from the group consisting of acetic acid or a salt thereof, phosphoric acid or a salt thereof, citric acid or a salt thereof, succinic acid or a salt thereof, glutamic acid or a salt thereof, 2-(N-morpholino)ethanesulfonic acid, and Tris or a salt thereof.

In one embodiment of the present invention, the concentration of the buffer (D) may be 0.1 to 50 mM.

In one embodiment of the present invention, the buffer (D) may include acetic acid or a salt thereof.

In one embodiment of the present invention, the antioxidant (E) may include at least one selected from the group consisting of methionine, ascorbic acid or a salt thereof, and taurine.

In one embodiment of the present invention, the antioxidant (E) may include methionine.

In one embodiment of the present invention, the concentration of the antioxidant (E) may be 0.1 to 100 mM.

In one embodiment of the present invention, the pH of the pharmaceutical formulation may be 4 to 7.

In one embodiment of the present invention, the pharmaceutical formulation may not include sucrose.

In one embodiment of the present invention, the pharmaceutical formulation may not include histidine.

In one embodiment of the present invention, the pharmaceutical formulation may be for intravenous administration or subcutaneous administration.

The present invention also provides a stable pharmaceutical formulation including: (A) 1 to 300 mg/ml of an antibody or antigen-binding fragment thereof including: a light-chain variable region including a CDR1 domain comprising the amino acid sequence of SEQ ID NO:1, a CDR2 domain comprising the amino acid sequence of SEQ ID NO: 2, and a CDR3 domain comprising the amino acid sequence of SEQ ID NO: 3; and a heavy-chain variable region including a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO:6; (B) 0.001 to 1% (w/v) of a surfactant; (C) 0.1 to 30% (w/v) of a sugar or sugar alcohol; (D) 0.1 to 50 mM of a buffer; and (E) 0.1 to 100 mM of an antioxidant.

In one embodiment of the present invention, the sugar or sugar alcohol (C) may include sorbitol, the buffer (D) may include acetic acid or a salt thereof, and the antioxidant (E) may include methionine.

The present invention also provides a vial filled with the stable pharmaceutical formulation according to the present invention.

The present invention also provides a cartridge filled with the stable pharmaceutical formulation according to the present invention.

The present invention also provides a pre-filled syringe filled with the stable pharmaceutical formulation according to the present invention.

The present invention also provides an auto-injector including the pre-filled syringe according to the present invention therein.

### Advantageous Effects

The stable pharmaceutical formulation according to the present invention has excellent long-term storage stability based on stability under accelerated conditions and harsh conditions, despite containing an antibody that binds to PD-1 (CD279, programmed cell death protein 1), and may be administered intravenously or subcutaneously.

### Mode for Invention

The present inventors have conducted repeated studies to overcome the above-described problems and, as a result, have developed a stable pharmaceutical formulation containing an antibody that binds to PD-1 (CD279, programmed cell death protein 1).

The present invention provides a stable pharmaceutical formulation including: (A) an antibody or antigen-binding fragment thereof that binds to PD-1 (CD279, programmed cell death protein 1); (B) a surfactant; (C) a stabilizer; (D) a buffer; and (E) an antioxidant.

In one embodiment of the present invention, the pharmaceutical formulation may be in a liquid state, without being limited thereto.

### (A) Antibody or Antigen-Binding Fragment Thereof

In the present invention, the term "antibody" refers to an immunoglobulin molecule consisting of four polypeptide chains configured such that two heavy chains and two light chains are linked to each other by disulfide bonds, and may include naturally occurring antibodies having other changed structures, for example, camelid antibodies.

Each heavy chain consists of a heavy-chain variable region and a heavy-chain constant region. The heavy-chain constant region consists of three domains (CH1, CH2 and CH3). Each light chain consists of a light-chain variable region and a light-chain constant region.

The light-chain constant region consists of one domain (CL). The heavy-chain variable region and the light-chain variable region may be further subdivided into regions of hypervariability, referred to as complementarity determining regions (CDRs), interspersed with regions that are more conserved, referred to as framework regions (FRs). Each of the heavy-chain variable region and the light-chain variable region consists of three CDRs and four FRs, which are arranged from the amino terminus to the carboxyl terminus in the following order: FR1, CDR1, FR2, CDR2, FR3, CDR3, and FR4.

In one embodiment of the present invention, the antibody or antigen-binding fragment according to the present invention may be a polyclonal antibody, a monoclonal antibody, a recombinant antibody, a single-chain antibody, a hybrid antibody, a chimeric antibody, a humanized antibody, or a fragment (antigen-binding fragment) thereof, preferably a monoclonal antibody or an antigen-binding fragment thereof.

The monoclonal antibody or antigen-binding fragment thereof may be a humanized monoclonal antibody or a fragment thereof, preferably a humanized immunoglobulin G (IgG) monoclonal antibody, and may be produced by a method known in the art.

In the present invention, the antigen-binding fragment may include any naturally occurring, enzymatically obtainable, synthetic, or genetically engineered polypeptide or glycoprotein that specifically binds to an antigen to form a complex, and may be, for example, Fab, F(ab'), F(ab')2, Fv, dAb, Fd, a single-chain antibody fragment (scFv), scFv-Fc, a complementarity-determining region (CDR) fragment, a bivalent single-chain antibody fragment, a single-chain phage antibody fragment, a diabody, a triabody, a tetrabody, or a nanobody, without being limited thereto.

In the present invention, the humanized antibody is also called a reshaped human antibody, may be obtained by transplanting the complementarity determining regions (CDRs) of a non-human mammal antibody, such as a mouse antibody, into the complementarity determining regions of a human antibody, and may also be produced by other generally known gene recombination techniques.

In the present invention, the antibody or antigen-binding fragment thereof may include: a light-chain variable region including a CDR1 domain comprising the amino acid sequence of SEQ ID NO:1, a CDR2 domain comprising the amino acid sequence of SEQ ID NO: 2, and a CDR3 domain comprising the amino acid sequence of SEQ ID NO: 3; and a heavy-chain variable region including a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

In the present invention, the antibody or antigen-binding fragment thereof may include a light-chain variable region comprising the amino acid sequence of SEQ ID NO: 7, and a heavy-chain variable region comprising the amino acid sequence of SEQ ID NO: 8.

In the present invention, the antibody or antigen-binding fragment thereof may include a light chain comprising the amino acid sequence of SEQ ID NO: 9, and a heavy chain comprising the amino acid sequence of SEQ ID NO: 10.

In the present invention, the antibody (A) may be pembrolizumab, nibolumab or a mixture thereof, preferably pembrolizumab.

In the present invention, the "pembrolizumab" may be an antibody against human programmed death receptor PD-1 described in International Patent Publication No. WO2012-135408, an originator drug substance well known in the art, or a biosimilar thereof.

In the present invention, the "nibolumab" may be a PD-1 (CD279, programmed cell death protein 1)-binding antibody, also known as Opdivo^{®}, or a biosimilar thereof.

In one embodiment of the present invention, the concentration of the antibody or antigen-binding fragment thereof (A) may be 1 to 300 mg/mL, preferably 5 to 200, more preferably 10 to 100, most preferably 15 to 35 mg/mL. When the concentration of the antibody or antigen-binding fragment thereof is within the above-described range, the freedom to determine the dose and frequency of administration may be increased, and long-term stability may be excellent.

### (B) Surfactant

In the present invention, the term "surfactant" refers to a substance that may be used to significantly increase the water solubility of a hydrophobic or oily substance or to increase the miscibility of two substances having different hydrophobicities.

In the present invention, the surfactant may be polyoxyethylene sorbitan fatty acid ester (e.g., polysorbate, etc.), polyoxyethylene alkyl ether (e.g., Brij^{®}, etc.), alkylphenylpolyoxyethylene ether (e.g., Triton-X, etc.), a polyoxyethylene-polyoxypropylene copolymer (e.g., poloxamer, Pluronic^{®}, etc.), sodium dodecyl sulfate (SDS), etc., without being limited thereto

In one embodiment of the present invention, the surfactant may include polysorbate, poloxamer, or a mixture thereof, and preferably may include polysorbate.

In the present invention, the polysorbate may include at least one selected from the group consisting of polysorbate 20, polysorbate 40, polysorbate 60, and polysorbate 80, and preferably may include polysorbate 80.

In one embodiment of the present invention, the concentration of the surfactant may be freely adjusted within a range that does not adversely affect the stability and viscosity of the stable liquid pharmaceutical formulation according to the present invention.

In one embodiment of the present invention, the concentration of the surfactant (B) may be 0.001 to 1% (w/v), preferably 0.005 to 0.5% (w/v), more preferably 0.01 to 0.1% (w/v), most preferably 0.015 to 0.05% (w/v). When the concentration of the surfactant is within the above-described range, long-term stability may be excellent.

### (C) Stabilizer

In the present invention, the term "stabilizer" is a substance that is physiologically acceptable and imparts stability to the formulation.

In one embodiment of the present invention, the stabilizer (C) may include at least one selected from the group consisting of i) an amino acid or amino acid derivative; and ii) a sugar or sugar alcohol.

In one embodiment of the present invention, the amino acid or amino acid derivative i) (provided that it is different from the component included as the buffer or antioxidant according to the present invention) may include at least one selected from the group consisting of threonine, arginine, proline, leucine, glycine, phenylalanine, tryptophan, glutamine, aspartate, glutamate, alanine, asparagine, serine, and tyrosine, and preferably include proline, without being limited thereto.

In one embodiment of the present invention, the sugar or sugar alcohol ii) may include at least one selected from the group consisting of trehalose, sorbitol, glucose, fructose, galactose, xylose, maltose, lactose, xylitol, mannitol, D-maltitol, inositol, lactitol, and isomalt, and preferably may include sorbitol, without being limited thereto.

In one embodiment of the present invention, the pharmaceutical formulation of the present invention may not include sucrose as a stabilizer in terms of long-term stability.

In the present invention, the concentration of the stabilizer may be freely adjusted within a range that does not substantially adversely affect the stability and viscosity of the pharmaceutical formulation according to the present invention.

In one embodiment of the present invention, when the stabilizer includes the amino acid or amino acid derivative i), the concentration of the amino acid or amino acid derivative may be 10 to 450 mM, preferably 100 to 350 mM, more preferably 150 to 300 mM, most preferably 180 to 270 mM.

In one embodiment of the present invention, when the stabilizer includes the sugar or sugar alcohol ii), the concentration of the sugar or sugar alcohol may be 0.1 to 30% (w/v), preferably 0.5 to 20% (w/v), more preferably 1 to 15% (w/v), most preferably 2 to 10% (w/v). When the concentration of the stabilizer is within the above-described range, long-term stability may be excellent.

### (D) Buffer

In the present invention, the term "buffer" refers to a neutralizing substance that minimizes a change in pH caused by an acid or an alkali

In one embodiment of the present invention, the buffer (D) may include at least one selected from the group consisting of acetic acid or a salt thereof, phosphoric acid or a salt thereof, citric acid or a salt thereof, succinic acid or a salt thereof, glutamic acid or a salt thereof, 2-(N-morpholino)ethanesulfonic acid (MES), tromethamine (Tris) or a salt thereof, and preferably may include acetic acid or a salt thereof, without being limited thereto.

The acetate may be, for example, sodium acetate, zinc acetate, aluminum acetate, ammonium acetate, potassium acetate, etc., without being limited thereto.

The phosphate may be, for example, potassium phosphate, sodium phosphate, ammonium phosphate, calcium phosphate, magnesium phosphate, etc., without being limited thereto.

The citrate may be, for example, sodium citrate, calcium citrate, potassium citrate, etc., without being limited thereto.

The succinate may be, for example, sodium succinate, calcium succinate, potassium succinate, sodium sulfosuccinate, potassium sulfosuccinate, calcium sulfosuccinate, etc., without being limited thereto.

The glutamate may be, for example, sodium glutamate, potassium glutamate, ammonium glutamate, etc., without being limited thereto.

The 2-(N-morpholino)ethanesulfonic acid (MES) salt may be, for example, MES chloride, MES sodium, etc., without being limited thereto.

The Tris salt may be, for example, Tris hydrochloride, Tris acetate, Tris borate, etc., without being limited thereto.

In one embodiment of the present invention, the pharmaceutical formulation of the present invention may not include histidine or a salt thereof as a buffer in terms of long-term stability.

In the present invention, the concentration of the buffer may be freely adjusted within a range that does not substantially adversely affect the stability and viscosity of the pharmaceutical formulation according to the present invention.

In one embodiment of the present invention, the concentration of the buffer (D) may be 0.1 to 50 mM, preferably 1 to 50 mM, more preferably 1 to 30 mM, most preferably 5 to 15 mM. When the concentration of the buffer is within the above-described range, long-term stability may be excellent.

### (E) Antioxidant

In the present invention, the term "antioxidant" refers to a substance that inhibits the oxidation-induced decrease in the activity of the antibody and the stability of the pharmaceutical formulation.

In one embodiment of the present invention, the antioxidant (E) may include at least one selected from the group consisting of methionine, ascorbic acid or a salt thereof, and taurine, and preferably may include methionine, without being limited thereto.

The salt of ascorbic acid may be, for example, sodium ascorbate, calcium ascorbate, potassium ascorbate, magnesium ascorbate, etc., without being limited thereto.

In one embodiment of the present invention, the concentration of the antioxidant may be 0.1 to 100 mM, preferably 10 to 80 mM, more preferably 35 to 70 mM, most preferably 55 to 65 mM. When the concentration of the antioxidant is within the above-described range, long-term stability may be excellent.

### (F) pH-Adjusting Agent

In the present invention, the term "pH-adjusting agent" refers to a substance that is used to adjust the pH of the stable pharmaceutical formulation.

The pH of the stable pharmaceutical formulation according to the present invention may be adjusted within a range that optimizes therapeutic efficacy using the pH-adjusting agent. In the present invention, the pH of the stable pharmaceutical formulation may be 4 to 7, preferably 5 to 6, more preferably 5.2 to 5.5, most preferably 5.2.

In one embodiment of the present invention, when a buffer is contained in a certain amount, the pH within the above range may be exhibited without a separate pH-adjusting agent. In one embodiment of the present invention, a separate pH adjusting agent (acid or base) may be additionally contained within a range that does not impair the stability of the pharmaceutical formulation and the antibody.

In the present invention, the content of the pH-adjusting agent may be freely adjusted within a range that does not substantially adversely affect the stability and viscosity of the pharmaceutical formulation according to the present invention.

In one embodiment of the present invention, the pH-adjusting agent (F) may be hydrochloric acid, acetic acid, phosphoric acid, sodium hydroxide, sodium bicarbonate, or the like, or a mixture thereof.

### (G) Other Components

In the present invention, the stable pharmaceutical formulation according to the present invention may not include a preservative. The preservative may be, for example, octadecyl dimethyl benzyl ammonium chloride, hexamethonium chloride, benzalkonium chloride, benzethonium chloride, phenol, butyl alcohol, benzyl alcohol, alkyl paraben, catechol, resorcinol, cyclohexanol, 3-pentanol, m-cresol, etc. If a preservative is included, it may have a negative effect on improving stability.

In the present specification, the term "not including" or "not include" means not including any component at all. In addition, the term means substantially not including any component, i.e., including any component within a range that does not affect the activity of the antibody or the stability or viscosity of the pharmaceutical formulation. For example, the term means including any component in an amount of 1 ppm (w/v) or less or 1 ppb (w/v) or less based on the total weight of the pharmaceutical formulation.

In the present invention, the stable pharmaceutical formulation of the present invention may further include an additive known in the art within a range that does not substantially adversely affect the stability and viscosity of the formulation. The additive may be, for example, an aqueous carrier, an antioxidant, or a mixture thereof. In the present invention, the aqueous carrier may be a carrier useful for the preparation of a pharmaceutical formulation, which is safe and non-toxic when administered to humans. Examples of the aqueous carrier include, but are not limited to, sterile water for injection (SWFI), bacteriostatic water for injection (BWFI), sterile saline solution, Ringer's solution, dextrose, and the like.

### (H) "Stable" Pharmaceutical Formulation

In the present invention, the term "stable" or "stabilization" means that the component according to the present invention or the composition or formulation including the same retains its physical stability, chemical stability and/or biological activity during the preparation process and/or during storage or preservation. In the present invention, various analytical techniques for measuring stability are readily available in the art.

In the present invention, the physical stability may be assessed by a method known in the art, such as measuring the apparent attenuation of light (absorbance or optical density) by a sample. This light attenuation measurement is related to the turbidity of the formulation. In addition, for physical stability, high-molecular-weight component content, low-molecular-weight component content, and intact protein content, etc. may be measured.

In one embodiment of the present invention, the term "stable" pharmaceutical formulation means a pharmaceutical formulation satisfying at least one of the following (H)-1 to (H)-7.

### (H)-1 HIC-HPLC Main Peak

Pharmaceutical formulations having:
- a main peak of 91% to 100% as measured by HIC-HPLC after storage for 3 days, 7 days, 2 weeks or 4 weeks at a temperature of 5±3°C;
- a main peak of 92% to 100% as measured by HIC-HPLC after storage for 18 months at a temperature of 5±3°C;
- a main peak of 91% to 100% as measured by HIC-HPLC after storage for 3 days, 7 days, 2 weeks or 4 weeks at a temperature of 40±2°C and a relative humidity of 75±5%;
- a main peak of 91% to 100% as measured by HIC-HPLC after storage for 3 days or 7 days at a temperature of 45°C;
- a main peak of 89% to 100% as measured by HIC-HPLC after storage for 72 hour at a temperature of 25±2°C and a relative humidity of 60±5% under 12,000 lux light illumination; and
- a main peak of 92% to 100% as measured by HIC-HPLC after storage for 5 hours with stirring at 1,000 RPM.

### (H)-2 HIC-HPLC Pre-Peak (Peak with Retention Time before Main Peak)

Pharmaceutical formulations having:
- a pre-peak of 0 to 5% as measured by HIC-HPLC after storage for 3 days, 7 days, 2 weeks or 4 weeks at a temperature of 5±3°C;
- a pre-peak of 0 to 5% as measured by HIC-HPLC after storage for 18 months at a temperature of 5±3°C;
- a pre-peak of 0 to 5% as measured by HIC-HPLC after storage for 3 days, 7 days, 2 weeks or 4 weeks at a temperature of 40±2°C and a relative humidity of 75±5%;
- a pre-peak of 0 to 5% as measured by HIC-HPLC after storage for 3 days or 7 days at a temperature of 45°C;
- a pre-peak of 0 to 8% as measured by HIC-HPLC after storage for 72 hours at a temperature of 25±2°C and a relative humidity of 60±5% under 12,000 lux light illumination; and
- a pre-peak of 0 to 5% as measured by HIC-HPLC after storage for 5 hours with stirring at 1,000 RPM.

### (H)-3 HIC-HPLC Post-Peak (Peak with Retention Time after Main Peak)

Pharmaceutical formulations having:
- a post-peak of 0 to 4% as measured by HIC-HPLC after storage for 3 days, 7 days, 2 weeks or 4 weeks at a temperature of 5±3°C;
- a post-peak of 0 to 4% as measured by HIC-HPLC after storage for 18 months at a temperature of 5±3°C;
- a post-peak of 0 to 5% as measured by HIC-HPLC after storage for 3 days, 7 days, 2 weeks or 4 weeks at a temperature of 40±2°C and a relative humidity of 75±5%;
- a post-peak of 0 to 5% as measured by HIC-HPLC after storage for 3 days or 7 days at a temperature of 45°C;
- a post-peak of 0 to 4% as measured by HIC-HPLC after storage for 72 hours at a temperature of 25±2°C and a relative humidity of 60±5% under 12,000 lux light illumination; and
- a pre-peak of 0 to 4% as measured by HIC-HPLC after storage for 5 hours with stirring at 1,000 RPM.

### (H)-4 SEC-HPLC Monomer (Main Peak)

Pharmaceutical formulations having:
- a monomer component of 99% to 100% as measured by SEC-HPLC after storage for 3 days, 7 days, 2 weeks or 4 weeks at a temperature of 5±3°C;
- a monomer component of 99% to 100% as measured by SEC-HPLC after storage for 18 months at a temperature of 5±3°C;
- a monomer component of 98% to 100% as measured by SEC-HPLC after storage for 3 days, 7 days, 2 weeks or 4 weeks at a temperature of 40±2°Cand a relative humidity of 75±5%;
- a monomer component of 98% to 100% as measured by SEC-HPLC after storage for 3 days or 7 days at a temperature of 45°C;
- a monomer component of 98% to 100% as measured by SEC-HPLC after storage for 72 hours at a temperature of 25±2°C and a relative humidity of 60±5% under 12,000 lux light illumination; and
- a monomer component of 99% to 100% as measured by SEC-HPLC after storage for 5 hours with stirring at 1,000 RPM.

### (H)-5 SEC-HPLC High-Molecular-Weight Component (Peak with Retention Time before Main Peak)

Pharmaceutical formulations having:
- a high-molecular-weight component of 0 to 1% as measured by SEC-HPLC after storage for 3 days, 7 days, 2 weeks or 4 weeks at a temperature of 5±3°C;
- a high-molecular-weight component of 0 to 1% as measured by SEC-HPLC after storage for 18 months at a temperature of 5±3°C;
- a high-molecular-weight component of 0 to 2% as measured by SEC-HPLC after storage for 3 days, 7 days, 2 weeks or 4 weeks at a temperature of 40±2°C and a relative humidity of 75±5%;
- a high-molecular-weight component of 0 to 2% as measured by SEC-HPLC after storage for 3 days or 7 days at a temperature of 45°C;
- a high-molecular-weight component of 0 to 2% as measured by SEC-HPLC after storage for 72 hours at a temperature of 25±2°C and a relative humidity of 60±5% under 12,000 lux light illumination; and
- a high-molecular-weight component of 0 to 1% as measured by SEC-HPLC after storage for 5 hours with stirring at 1,000 RPM.

### (H)-6 SEC-HPLC Low-Molecular-Weight Component (Peak with Retention Time after Main Peak)

Pharmaceutical formulations having:
- a low-molecular-weight component of 0 to 1% as measured by SEC-HPLC after storage for 3 days, 7 days, 2 weeks or 4 weeks at a temperature of 5±3°C;
- a low-molecular-weight component of 0 to 1% as measured by SEC-HPLC after storage for 18 months at a temperature of 5±3°C;
- a low-molecular-weight component of 0 to 1% as measured by SEC-HPLC after storage for 3 days, 7 days, 2 weeks or 4 weeks at a temperature of 40±2°C and a relative humidity of 75±5%;
- a low-molecular-weight component of 0 to 1% as measured by SEC-HPLC after storage for 3 days or 7 days at a temperature of 45°C;
- a low-molecular-weight component of 0 to 1% as measured by SEC-HPLC after storage for 72 hours at a temperature of 25±2°C and a relative humidity of 60±5% under 12,000 lux light illumination; and
- a low-molecular-weight component of 0 to 1% as measured by SEC-HPLC after storage for 5 hours with stirring at 1,000 RPM.

### (H)-7 Methionine Oxidation Rate

Pharmaceutical formulations having:
- a heavy-chain Met105 oxidation rate of 0 to 2% and a heavy-chain Met252 oxidation rate of 0 to 5%, as measured by LC/MS after storage for 3 days, 7 days or 4 weeks at a temperature of 5±3°C;
- a heavy-chain Met105 oxidation rate of 0 to 2% and a heavy-chain Met252 oxidation rate of 0 to 5%, as measured by LC/MS after storage for 18 months at a temperature of 5±3°C;
- a heavy-chain Met105 oxidation rate of 0 to 2% and a heavy-chain Met252 oxidation rate of 0 to 5%, as measured by LC/MS after storage for 3 days, 7 days or 4 weeks at a temperature of 40±2°C and a relative temperature of 75±5%;
- a heavy-chain Met105 oxidation rate of 0 to 2% and a heavy-chain Met252 oxidation rate of 0 to 5%, as measured by LC/MS after storage for 3 days or 7 days at a temperature of 45°C;
- a heavy-chain Met105 oxidation rate of 0 to 2% and a heavy-chain Met252 oxidation rate of 0 to 10%, as measured by LC/MS after storage for 72 hours at a temperature of 25±2°C and a relative humidity of 60±5% under 12,000 lux light illumination; and
- a heavy-chain Met105 oxidation rate of 0 to 1% and a heavy-chain Met252 oxidation rate of 0 to 4%, as measured by LC/MS after storage for 5 hours with stirring at 1,000 RPM.

### [Product]

The present invention provides a product including: the stable pharmaceutical formulation; and a container that accommodates the stable pharmaceutical formulation in an airtight state.

The stable pharmaceutical formulation is as described above in the present specification.

In the present invention, the container may be formed of a material such as glass, a polymer (e.g., plastic), a metal, or the like, without being limited thereto.

For example, the container according to the present invention may be a bottle, a vial, a cartridge, a syringe (e.g., a pre-filled syringe, an auto-injector, etc.), or a tube, preferably a vial made of glass or a polymer, or a pre-filled syringe made of glass or a polymer, without being limited thereto.

In one embodiment of the present invention, the present invention provides a vial filled with the stable pharmaceutical formulation according to the present invention, a cartridge filled with the stable pharmaceutical formulation, a pre-filled syringe filled with the stable pharmaceutical formulation, or an auto-injector including the pre-filled syringe therein.

Specific product types of the vial, cartridge, pre-filled syringe, auto-injector, etc., and the method of filling the vial, cartridge, pre-filled syringe, auto-injector, etc. with the stable pharmaceutical formulation may be easily obtained or implemented by those of ordinary skill in the art to which the present invention pertains. For example, U.S. Pat. Nos. 4,861,335, 6,331,174 and the like disclose specific product types and filling methods of pre-filled syringes. For example, U.S. Pat. Nos. 5,085,642, 5,681,291 and the like disclose specific product types and assembly methods of auto-injectors. As the vial, cartridge, pre-filled syringe, auto-injector, etc., a commercially available product may be used without change, or a separately custom-made product may be used in consideration of the physical properties, administration site, dose and the like of the stable pharmaceutical formulation.

In the present invention, the container may be a single-dose container.

In the present invention, the product according to the present invention may additionally include instructions providing a method of using the stable pharmaceutical formulation, a method of storing the stable pharmaceutical formulation, or both. The instructions may include a treatment method for a PD-1-related disease, the route of administration, dose, or administration time.

In the present invention, the product may include other tools necessary from the viewpoint of a commercial purpose and a user, for example, a needle, a syringe, and the like.

### [Method for Preparing Stable Pharmaceutical Formulation]

The stable pharmaceutical formulation of the present invention may be prepared by a known method, and is not limited to a specific preparation method. For example, the pharmaceutical formulation may be prepared by adjusting the pH by adding a buffer to a solution containing a surfactant, a stabilizer and an antioxidant, and then adding an antibody to the mixed solution. As another example, the pharmaceutical formulation may be prepared by preparing a solution including an antibody, a buffer, a stabilizer and an antioxidant in the final step of the purification process, and then adding a surfactant to the solution.

### [Method of Using Stable Pharmaceutical Formulation]

In the present invention, the stable pharmaceutical formulation according to the present invention may exhibit a therapeutic effect on a disease associated with PD-1 (CD279, programmed cell death protein 1).

In the present invention, the stable pharmaceutical formulation according to the present invention may be used for intravenous (IV) administration or subcutaneous (SC) administration.

In one embodiment of the present invention, the stable pharmaceutical formulation may not undergo a reconstitution step prior to use.

The concentrations of other components including the antibody in the pharmaceutical formulation are as described above, and the total volume of the pharmaceutical formulation according to the present invention may be 0.5 to 20.0 mL.

The dose or administration time of the pharmaceutical formulation may vary depending on the type of disease, the severity and progress of the disease, the patient's health and response to treatment, or the judgment of the treating physician, and is not limited to a specific dose or administration time.

### [Treatment Method and Stabilization Method]

In one embodiment of the present invention, the present invention provides a method for treating a disease whose symptoms are effectively alleviated by inhibition of the binding of PD-L1 (CD274, programmed cell death-ligand 1) and PD-L2 (CD273, programmed cell death-ligand 2) to PD-1 (CD279, programmed cell death protein 1), the method including administering, to a patient having the disease, a stable pharmaceutical formulation including (A) an antibody or antigen-binding fragment thereof that binds to PD-1 (CD279, programmed cell death protein 1), (B) a surfactant, (C) a stabilizer, (D) a buffer, and (E) an antioxidant.

In another embodiment of the present invention, the present invention provides a method of stabilizing an antibody in a pharmaceutical formulation, including preparing a stable pharmaceutical formulation including (A) an antibody or antigen-binding fragment thereof that binds to PD-1 (CD279, programmed cell death protein 1), (B) a surfactant, (C) a stabilizer, (D) a buffer, and (E) an antioxidant.

The stable pharmaceutical formulation is as described above in the present specification.

For the treatment method or stabilization method according to the present invention, the above description in the present specification applies.

The above-described individual features described herein may be used in combination, and the fact that the above-described individual features are set forth in different dependent claims of the appended claims does not indicate that they cannot be used in combination.

Hereinafter, the present invention will be described in detail by way of examples. The following examples are only intended to illustrate the present invention, and the scope of the present invention is not limited by the following examples. The documents referred to in the present invention are incorporated by reference into the specification of the present invention.

In the following examples and comparative examples, pembrolizumab incubated and purified at the Celltrion Research Institute was used. The amino acid sequence of pembrolizumab used in the examples and comparative examples herein is as shown in SEQ ID NOs: 1 to 10 attached to the present specification.

In addition, the physical stability, chemical stability, and biological activity of the pharmaceutical formulations described below were measured using the following methods.

### - HIC-HPLC Main Peak

Main peak (%) was measured using hydrophobic interaction high-performance liquid chromatography (HIC-HPLC).

### - HIC-HPLC Pre-Peak

Pre-peak (%) was measured using hydrophobic interaction high-performance liquid chromatography (HIC-HPLC).

### - HIC-HPLC Post-Peak

Post-peak (%) was measured using hydrophobic interaction high-performance liquid chromatography (HIC-HPLC).

### - SEC-HPLC Main Component

Monomer (%) was measured using size-exclusion high-performance liquid chromatography (SEC-HPLC).

### - SEC-HPLC High-Molecular-Weight Component

High-molecular-weight ((HMW) component (%) was measured using size-exclusion high-performance liquid chromatography (SEC-HPLC).

### - SEC-HPLC Low-Molecular-Weight Component

Low-molecular-weight (LMW) component (%) was measured using size-exclusion high-performance liquid chromatography (SEC-HPLC).

### - Methionine Oxidation Rate

The heavy-chain Met105 and Met252 oxidation rates (%) of pembrolizumab were measured by peptide mapping using liquid chromatography-mass spectrometry (LC-MS).

### Examples 1 to 5 and Comparative Examples 1 to 3: Preparation of Pharmaceutical Formulations Containing Antibody That Binds to PD-1 (CD279, programmed cell death protein 1)

Examples 1 to 5 in Table 1 below were prepared by preparing each buffer according to each pH,and then adding a stabilizer and an antioxidant thereto, followed by the addition of an antibody and then a surfactant. Comparative Examples 1 to 3 were prepared by applying the same formulation as the originally developed pharmaceutical product to the same antibody as in the Examples, and were analyzed for comparison with each Example. The specific composition of components is shown in Table 1 below.

**[Table 1]**

| | Antibody | Surfactant | Stabilizer | Antioxidant | Buffer | pH | Container |
|---|---|---|---|---|---|---|---|
| **Example 1** | 25 mg/mL | Polysorbate 80 0.02% (w/v) | Sorbitol 4.5% | Methionine 35 mM | Acetate 10 mM | 5.5 | Vial |
| **Example 2** | 25 mg/mL | Polysorbate 80 0.02% (w/v) | Sorbitol 4.5% | Methionine 35 mM | Acetate 10 mM | 5.2 | Vial |
| **Example 3** | 25 mg/mL | Polysorbate 80 0.02% (w/v) | Sorbitol 4% | Methionine 60 mM | Acetate 10 mM | 5.5 | Vial |
| **Example 4** | 25 mg/mL | Polysorbate 80 0.02% (w/v) | Sorbitol 4% | Methionine 60 mM | Acetate 10 mM | 5.2 | Vial |
| **Example 5** | 25 mg/mL | Polysorbate 80 0.02% (w/v) | Proline 217 mM | Methionine 35 mM | Acetate 10 mM | 5.2 | Vial |
| **Comparative Example 1** | 25 mg/mL | Polysorbate 80 0.02% (w/v) | Sorbitol 5% | - | Acetate 10 mM | 5.5 | Vial |
| **Comparative Example 2** | 25 mg/mL | Polysorbate 80 0.02% (w/v) | Sorbitol 5% | - | Acetate 10 mM | 5.2 | Vial |
| **Comparative Example 3** | 25 mg/mL | Polysorbate 80 0.02% (w/v) | Sucrose 7% | - | Histidine 10 mM | 5.5 | Vial |

### Experimental Example: Stability Evaluation

Examples 1 to 5 and Comparative Examples 1 to 3 prepared above were stored under conditions of 5±3°C temperature; 40±2°C temperature and 75±5% relative humidity; 45°C temperature; 12,000 lux light illumination, 25±2°C temperature, 60±5% relative humidity, and 72 hours; and stirring at 1,000 RPM and 5 hours, and then the stability thereof for each condition was evaluated using the above-described method. The results are shown in Table 2 below.

In addition, the batches of Examples 1-1 and 1-2 prepared as described below were measured for their stability after 0 months, 6 months, 12 months, and 18 months at a temperature of 5±3°C, and the results are shown in Table 3 below.

**[Table2]**

| | | Example | | | | | Comparative Example | | |
|---|---|---|---|---|---|---|---|---|---|
| | | 1 | 2 | 3 | 4 | 5 | 1 | 2 | 3 |
| Antioxidant: methionine (mM) | | 35 | 35 | 60 | 60 | 35 | - | - | - |
| Antibody: pembrolizumab (mg/ml) | | 25 | 25 | 25 | 25 | 25 | 25 | 25 | 25 |
| Buffer | Acetate (mM) | 10 | 10 | 10 | 10 | 10 | 10 | 10 | - |
| | Histidine (mM) | - | - | - | - | - | - | - | 10 |
| Surfactant: polysorbate 80 (%, w/v) | | 0.02 | 0.02 | 0.02 | 0.02 | 0.02 | 0.02 | 0.02 | 0.02 |
| Stabilizer | Sorbitol (%, w/v) | 4.5 | 4.5 | 4 | 4 | - | 5 | 5 | - |
| | Proline (mM) | - | - | - | - | 217 | - | - | - |
| | Sucrose (%, w/v) | - | - | - | - | - | - | - | 7 |
| pH | | 5.5 | 5.2 | 5.5 | 5.2 | 5.2 | 5.5 | 5.2 | 5.5 |
| 3 days at 5±3°C temperature | HIC-HPLC main peak (%) | 92.72 | 92.84 | 92.85 | 92.79 | 92.85 | 92.70 | 92.37 | 92.63 |
| | HIC-HPLC pre-peak (%) | 3.84 | 3.84 | 3.89 | 3.85 | 3.84 | 4.03 | 4.21 | 4.05 |
| | HIC-HPLC post-peak (%) | 3.44 | 3.32 | 3.26 | 3.36 | 3.31 | 3.27 | 3.41 | 3.32 |
| | SEC-HPLC monomer (%) | 99.56 | 99.61 | 99.50 | 99.54 | 99.54 | 99.51 | 99.49 | 99.52 |
| | SEC-HPLC high-molecular-weight component (%) | 0.39 | 0.36 | 0.44 | 0.43 | 0.40 | 0.45 | 0.45 | 0.46 |
| | SEC-HPLC low-molecular-weight component (%) | 0.05 | 0.03 | 0.06 | 0.04 | 0.05 | 0.04 | 0.06 | 0.02 |
| | Heavy-chain Met105 oxidation rate (%) | 1.9 | 1.4 | 1.9 | 1.8 | 1.8 | 1.9 | 2.0 | 1.9 |
| | Heavy-chain Met252 oxidation rate (%) | 4.3 | 3.5 | 3.5 | 3.9 | 4.0 | 4.7 | 4.7 | 3.7 |
| 7 days at 5±3°C temperature | HIC-HPLC main peak (%) | 91.94 | 92.00 | 92.11 | 92.01 | 92.26 | 91.20 | 91.15 | 90.97 |
| | HIC-HPLC pre-peak (%) | 4.11 | 4.37 | 4.24 | 4.21 | 4.07 | 4.77 | 4.84 | 4.72 |
| | HIC-HPLC post-peak (%) | 3.94 | 3.50 | 3.65 | 3.79 | 3.67 | 4.03 | 4.01 | 4.31 |
| | SEC-HPLC monomer (%) | 99.55 | 99.57 | 99.55 | 99.62 | 99.59 | 99.41 | 99.46 | 99.54 |
| | SEC-HPLC high-molecular-weight component (%) | 0.42 | 0.41 | 0.43 | 0.36 | 0.39 | 0.55 | 0.52 | 0.44 |
| | SEC-HPLC low-molecular-weight component (%) | 0.03 | 0.02 | 0.02 | 0.01 | 0.02 | 0.03 | 0.03 | 0.02 |
| | Heavy-chain Met105 oxidation rate (%) | 1.4 | 1.4 | 1.3 | 1 | 1.2 | 1.7 | 1.5 | 1.4 |
| | Heavy-chain Met252 oxidation rate (%) | 3.7 | 3.8 | 4.0 | 3.9 | 3.8 | 4.1 | 3.9 | 3.9 |
| 2 weeks at 5±3°C temperature | HIC-HPLC main peak (%) | 92.96 | 93.08 | 92.94 | 92.94 | 92.94 | 92.88 | 92.76 | 91.97 |
| | HIC-HPLC pre-peak (%) | 4.01 | 3.91 | 4.02 | 3.95 | 4.03 | 4.28 | 4.32 | 5.06 |
| | HIC-HPLC post-peak (%) | 3.03 | 3.01 | 3.04 | 3.11 | 3.04 | 2.84 | 2.92 | 2.96 |
| | SEC-HPLC monomer (%) | 99.42 | 99.47 | 99.47 | 99.55 | 99.47 | 99.41 | 99.46 | 99.48 |
| | SEC-HPLC high-molecular-weight component (%) | 0.51 | 0.50 | 0.49 | 0.41 | 0.48 | 0.55 | 0.47 | 0.48 |
| | SEC-HPLC low-molecular-weight component (%) | 0.07 | 0.03 | 0.05 | 0.04 | 0.06 | 0.04 | 0.07 | 0.03 |
| | Heavy-chain Met105 oxidation rate (%) | - | - | - | - | - | - | - | - |
| | Heavy-chain Met252 oxidation rate (%) | - | - | - | - | - | - | - | - |
| 4 weeks at 5±3°C temperature | HIC-HPLC main peak (%) | 92.74 | 92.79 | 92.74 | 92.96 | 92.90 | 92.25 | 92.60 | 90.95 |
| | HIC-HPLC pre-peak (%) | 3.84 | 3.86 | 3.84 | 3.79 | 3.89 | 4.35 | 4.21 | 5.42 |
| | HIC-HPLC post-peak (%) | 3.41 | 3.35 | 3.42 | 3.24 | 3.21 | 3.40 | 3.19 | 3.63 |
| | SEC-HPLC monomer (%) | 99.50 | 99.62 | 99.42 | 99.57 | 99.59 | 99.46 | 99.61 | 99.49 |
| | SEC-HPLC high-molecular-weight component (%) | 0.47 | 0.35 | 0.48 | 0.37 | 0.37 | 0.50 | 0.34 | 0.46 |
| | SEC-HPLC low-molecular-weight component (%) | 0.03 | 0.03 | 0.10 | 0.06 | 0.03 | 0.04 | 0.05 | 0.05 |
| | Heavy-chain Met105 oxidation rate (%) | 1.9 | 1.9 | 1.8 | 1.8 | 1.9 | 2.3 | 2.2 | 2.6 |
| | Heavy-chain Met252 oxidation rate (%) | 4.4 | 4.5 | 4.4 | 4.4 | 4.4 | 4.6 | 4.6 | 4.7 |
| 3 days at 40±2°C temperature and 75±5% relative humidity | HIC-HPLC main peak (%) | 92.70 | 92.75 | 92.78 | 92.90 | 92.85 | 91.78 | 92.11 | 91.22 |
| | HIC-HPLC pre-peak (%) | 3.94 | 3.92 | 3.81 | 3.81 | 3.82 | 4.63 | 4.60 | 5.22 |
| | HIC-HPLC post-peak (%) | 3.36 | 3.34 | 3.41 | 3.29 | 3.33 | 3.60 | 3.28 | 3.56 |
| | SEC-HPLC monomer (%) | 99.47 | 99.47 | 99.50 | 99.53 | 99.50 | 99.39 | 99.39 | 99.53 |
| | SEC-HPLC high-molecular-weight component (%) | 0.50 | 0.48 | 0.44 | 0.42 | 0.47 | 0.58 | 0.55 | 0.42 |
| | SEC-HPLC low-molecular-weight component (%) | 0.03 | 0.05 | 0.06 | 0.05 | 0.03 | 0.03 | 0.06 | 0.05 |
| | Heavy-chain Met105 oxidation rate (%) | 1.7 | 1.7 | 1.7 | 1.3 | 1.1 | 1.9 | 1.8 | 1.8 |
| | Heavy-chain Met252 oxidation rate (%) | 3.7 | 3.5 | 3.7 | 4.1 | 2.6 | 3.9 | 4.0 | 4.4 |
| 7 days at 40±2°C temperature and 75±5% relative humidity | HIC-HPLC main peak (%) | 91.37 | 91.45 | 91.86 | 91.83 | 91.47 | 90.43 | 90.47 | 90.11 |
| | HIC-HPLC pre-peak (%) | 4.56 | 4.31 | 4.32 | 4.33 | 4.52 | 5.47 | 5.68 | 5.91 |
| | HIC-HPLC post-peak (%) | 4.07 | 4.24 | 3.83 | 3.84 | 4.01 | 4.11 | 3.86 | 3.98 |
| | SEC-HPLC monomer (%) | 99.27 | 99.35 | 99.30 | 99.32 | 99.26 | 99.04 | 99.22 | 99.29 |
| | SEC-HPLC high-molecular-weight component (%) | 0.69 | 0.61 | 0.66 | 0.63 | 0.70 | 0.92 | 0.75 | 0.68 |
| | SEC-HPLC low-molecular-weight component (%) | 0.04 | 0.04 | 0.03 | 0.05 | 0.04 | 0.04 | 0.04 | 0.04 |
| | Heavy-chain Met105 oxidation rate (%) | 1.3 | 1.2 | 1.0 | 1.0 | 1.0 | 1.8 | 1.9 | 1.8 |
| | Heavy-chain Met252 oxidation rate (%) | 3.9 | 3.9 | 3.7 | 3.9 | 3.9 | 4.4 | 4.3 | 4.3 |
| 2 weeks at 40±2°C temperature and 75±5% relative humidity | HIC-HPLC main peak (%) | 92.89 | 92.76 | 93.06 | 92.90 | 92.97 | 91.67 | 91.34 | 90.30 |
| | HIC-HPLC pre-peak (%) | 3.96 | 3.96 | 3.84 | 3.83 | 3.91 | 5.43 | 5.49 | 6.68 |
| | HIC-HPLC post-peak (%) | 3.14 | 3.28 | 3.10 | 3.27 | 3.12 | 2.90 | 3.16 | 3.02 |
| | SEC-HPLC monomer (%) | 99.01 | 99.12 | 98.99 | 99.15 | 99.12 | 98.54 | 98.94 | 99.04 |
| | SEC-HPLC high-molecular-weight component (%) | 0.91 | 0.82 | 0.97 | 0.79 | 0.82 | 1.38 | 1.00 | 0.90 |
| | SEC-HPLC low-molecular-weight component (%) | 0.08 | 0.06 | 0.04 | 0.07 | 0.06 | 0.08 | 0.05 | 0.06 |
| | Heavy-chain Met105 oxidation rate (%) | - | - | - | - | - | - | - | - |
| | Heavy-chain Met252 oxidation rate (%) | - | - | - | - | - | - | - | - |
| 4 weeks at 40±2°C temperature and 75±5% | HIC-HPLC main peak (%) | 91.81 | 92.02 | 92.09 | 92.07 | 92.05 | 89.42 | 89.68 | 86.56 |
| | HIC-HPLC pre-peak (%) | 4.05 | 4.05 | 4.03 | 3.94 | 4.08 | 6.29 | 6.21 | 9.51 |
| | HIC-HPLC post-peak (%) | 4.14 | 3.94 | 3.88 | 3.98 | 3.86 | 4.30 | 4.11 | 3.93 |
| | SEC-HPLC monomer (%) | 98.72 | 98.74 | 98.79 | 98.93 | 98.79 | 98.19 | 98.55 | 98.71 |
| relative humidity | SEC-HPLC high-molecular-weight component (%) | 1.17 | 1.15 | 1.10 | 0.98 | 1.13 | 1.71 | 1.36 | 1.24 |
| | SEC-HPLC low-molecular-weight component (%) | 0.11 | 0.11 | 0.11 | 0.09 | 0.08 | 0.10 | 0.09 | 0.05 |
| | Heavy-chain Met105 oxidation rate (%) | 1.7 | 1.7 | 1.5 | 1.4 | 1.5 | 2.8 | 2.8 | 3.9 |
| | Heavy-chain Met252 oxidation rate (%) | 4.3 | 4.3 | 4.2 | 4.3 | 4.3 | 5.1 | 4.9 | 5.6 |
| 3 days at 45°C temperature | HIC-HPLC main peak (%) | 92.79 | 92.81 | 92.90 | 93.04 | 92.63 | 91.72 | 91.83 | 91.31 |
| | HIC-HPLC pre-peak (%) | 3.82 | 3.80 | 3.87 | 3.73 | 3.90 | 4.86 | 4.75 | 5.20 |
| | HIC-HPLC post-peak (%) | 3.39 | 3.40 | 3.24 | 3.23 | 3.47 | 3.42 | 3.40 | 3.50 |
| | SEC-HPLC monomer (%) | 99.02 | 99.03 | 98.95 | 99.17 | 99.06 | 98.91 | 98.85 | 99.08 |
| | SEC-HPLC high-molecular-weight component (%) | 0.91 | 0.86 | 0.96 | 0.74 | 0.90 | 1.07 | 1.05 | 0.86 |
| | SEC-HPLC low-molecular-weight component (%) | 0.07 | 0.10 | 0.10 | 0.09 | 0.04 | 0.02 | 0.10 | 0.05 |
| | Heavy-chain Met105 oxidation rate (%) | 1.5 | 1.4 | 1.4 | 1.0 | 1.3 | 1.8 | 1.4 | 1.8 |
| | Heavy-chain Met252 oxidation rate (%) | 4.1 | 4.1 | 4.3 | 4.2 | 4.4 | 4.1 | 4.1 | 4.4 |
| 7 days at 45°C temperature | HIC-HPLC main peak (%) | 91.47 | 91.88 | 91.24 | 92.33 | 91.62 | 90.30 | 89.34 | 88.83 |
| | HIC-HPLC pre-peak (%) | 4.50 | 4.37 | 4.28 | 4.03 | 4.29 | 5.68 | 6.00 | 6.62 |
| | HIC-HPLC post-peak (%) | 4.02 | 3.75 | 4.48 | 3.64 | 4.10 | 4.02 | 4.66 | 4.54 |
| | SEC-HPLC monomer (%) | 98.53 | 98.59 | 98.69 | 98.72 | 98.57 | 98.43 | 98.58 | 98.69 |
| | SEC-HPLC high-molecular-weight component (%) | 1.41 | 1.35 | 1.25 | 1.22 | 1.35 | 1.51 | 1.36 | 1.26 |
| | SEC-HPLC low-molecular-weight component (%) | 0.07 | 0.05 | 0.06 | 0.06 | 0.07 | 0.06 | 0.06 | 0.05 |
| | Heavy-chain Met105 oxidation rate (%) | 1.1 | 1.0 | 1.1 | 1.1 | 1.0 | 1.1 | 1.5 | 1.7 |
| | Heavy-chain Met252 oxidation rate (%) | 3.3 | 3.9 | 3.6 | 3.6 | 3.5 | 0.0 | 4.2 | 4.4 |
| 72 hours at 25±2°C temperature and 60±5% relative humidity under 12,000 lux light illumination | HIC-HPLC main peak (%) | 89.60 | 89.70 | 90.38 | 90.42 | 89.28 | 82.34 | 81.46 | 80.78 |
| | HIC-HPLC pre-peak (%) | 6.74 | 6.75 | 6.08 | 6.05 | 7.05 | 14.03 | 15.52 | 16.13 |
| | HIC-HPLC post-peak (%) | 3.67 | 3.54 | 3.55 | 3.54 | 3.68 | 3.63 | 3.03 | 3.08 |
| | SEC-HPLC monomer (%) | 98.63 | 98.74 | 98.71 | 98.87 | 98.65 | 97.59 | 98.28 | 98.22 |
| | SEC-HPLC high-molecular-weight component (%) | 1.32 | 1.19 | 1.26 | 1.06 | 1.30 | 2.34 | 1.66 | 1.72 |
| | SEC-HPLC low-molecular-weight component (%) | 0.04 | 0.07 | 0.03 | 0.07 | 0.06 | 0.07 | 0.06 | 0.06 |
| | Heavy-chain Met105 oxidation rate (%) | 1.3 | 1.6 | 0.9 | 0.8 | 1.5 | 4.2 | 5.0 | 4.4 |
| | Heavy-chain Met252 oxidation rate (%) | 8.7 | 9.3 | 5.9 | 6.1 | 8.3 | 17.0 | 18.1 | 13.4 |
| | HIC-HPLC main peak (%) | 92.39 | 92.32 | 92.37 | 92.68 | 92.14 | 89.60 | 92.20 | 92.26 |
| | HIC-HPLC pre-peak (%) | 4.07 | 4.11 | 3.99 | 3.95 | 4.13 | 4.83 | 4.44 | 4.33 |
| | HIC-HPLC post-peak (%) | 3.55 | 3.56 | 3.64 | 3.38 | 3.73 | 5.56 | 3.36 | 3.41 |
| | SEC-HPLC monomer (%) | 99.52 | 99.59 | 99.52 | 99.62 | 99.58 | 99.46 | 99.53 | 99.54 |
| 5 hours with stirring at 1,000 RPM | SEC-HPLC high-molecular-weight component (%) | 0.44 | 0.38 | 0.44 | 0.35 | 0.40 | 0.51 | 0.45 | 0.42 |
| | SEC-HPLC low-molecular-weight component (%) | 0.04 | 0.03 | 0.03 | 0.03 | 0.02 | 0.03 | 0.02 | 0.03 |
| | Heavy-chain Met105 oxidation rate (%) | 0.8 | 0.9 | 0.8 | 0.7 | 0.9 | 1.2 | 1.2 | 1.0 |
| | Heavy-chain Met252 oxidation rate (%) | 3.7 | 3.2 | 3.8 | 3.6 | 3.5 | 2.9 | 3.7 | 3.5 |

**[Table 3]**

| | | Example 1-1 | Example 1-2 |
|---|---|---|---|
| Antioxidant: methionine (mM) | | 60 | |
| Antibody: pembrolizumab (mg/ml) | | 25 | |
| Buffer: acetate (mM) | | 10 | |
| Surfactant: polysorbate 80 (%, w/v) | | 0.02 | |
| Stabilizer: sorbitol (%, w/v) | | 4 | |
| pH | | 5.2 | |
| After 0 months at 5±3°C temperature | HIC-HPLC main peak (%) | 93.42 | 93.82 |
| | HIC-HPLC pre-peak (%) | 3.77 | 3.50 |
| | HIC-HPLC post-peak (%) | 2.82 | 2.68 |
| | SEC-HPLC monomer (%) | 99.80 | 99.83 |
| | SEC-HPLC high-molecular-weight component (%) | 0.20 | 0.17 |
| | SEC-HPLC low-molecular-weight component (%) | 0.00 | 0.00 |
| | Heavy-chain Met105 oxidation rate (%) | 1.6 | 1.6 |
| | Heavy-chain Met252 oxidation rate (%) | 3.0 | 3.2 |
| After 6 months at 5±3°C temperature | HIC-HPLC main peak (%) | 93.11 | 93.31 |
| | HIC-HPLC pre-peak (%) | 3.91 | 3.97 |
| | HIC-HPLC post-peak (%) | 2.98 | 2.71 |
| | SEC-HPLC monomer (%) | 99.74 | 99.75 |
| | SEC-HPLC high-molecular-weight component (%) | 0.26 | 0.25 |
| | SEC-HPLC low-molecular-weight component (%) | 0.00 | 0.00 |
| | Heavy-chain Met105 oxidation rate (%) | 1.6 | 1.4 |
| | Heavy-chain Met252 oxidation rate (%) | 2.2 | 2.6 |
| After 12 months at 5±3°C temperature | HIC-HPLC main peak (%) | 93.13 | 93.37 |
| | HIC-HPLC pre-peak (%) | 3.55 | 3.61 |
| | HIC-HPLC post-peak (%) | 3.32 | 3.02 |
| | SEC-HPLC monomer (%) | 99.72 | 99.72 |
| | SEC-HPLC high-molecular-weight component (%) | 0.28 | 0.28 |
| | SEC-HPLC low-molecular-weight component (%) | 0.00 | 0.00 |
| | Heavy-chain Met105 oxidation rate (%) | 1.9 | 1.8 |
| | Heavy-chain Met252 oxidation rate (%) | 2.9 | 3.0 |
| | HIC-HPLC main peak (%) | 93.24 | 93.40 |
| | HIC-HPLC pre-peak (%) | 3.78 | 3.81 |
| | HIC-HPLC post-peak (%) | 2.97 | 2.80 |
| After 18 months at 5±3°C temperature | SEC-HPLC monomer (%) | 99.70 | 99.69 |
| | SEC-HPLC high-molecular-weight component (%) | 0.30 | 0.31 |
| | SEC-HPLC low-molecular-weight component (%) | 0.00 | 0.00 |
| | Heavy-chain Met105 oxidation rate (%) | 2.0 | 1.9 |
| | Heavy-chain Met252 oxidation rate (%) | 2.8 | 2.8 |

### (1) Comparison of Stability Depending on Antioxidant Content in pH 5.5 Formulation

From the results in Table 2 above, it could be seen that, under the condition of temperature of 5±3°C, both Examples 1 and 3 had an HIC-HPLC main peak of 91% or more, an HIC-HPLC pre-peak of 5% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the conditions of temperature of 40±2°C and relative humidity of 75±5%, both Examples 1 and 3 had an HIC-HPLC main peak of 91% or more, an HIC-HPLC pre-peak of 5% or less, an HIC-HPLC post-peak of 5% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the condition of temperature of 45°C, both Examples 1 and 3 had an HIC-HPLC main peak of 91% or more, an HIC-HPLC pre-peak of 5% or less, an HIC-HPLC post-peak of 5% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the conditions of 12,000 lux light illumination, temperature of 25±2°C, relative humidity of 60±5%, and storage time of 72 hours, both Examples 1 and 3 had an HIC-HPLC main peak of 89% or more, an HIC-HPLC pre-peak of 8% or less, an HIC-HPLC post-peak of 4% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 10% or less, indicating that they were pharmaceutically acceptable stable examples.

In addition, it could be seen that, under the conditions of stirring at 1,000 RPM and storage time of 5 hours, both Examples 1 and 3 had an HIC-HPLC main peak of 92% or more, an HIC-HPLC pre-peak of 5% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, a heavy-chain Met105 oxidation rate of 1% or less, and a heavy-chain Met252 oxidation rate of 4% or less, indicating that they were pharmaceutically acceptable stable examples.

In addition, through comparison of Examples 1 and 3 with Comparative Example 1, it was confirmed that, as the content of methionine increased, the HIC-HPLC main peak was higher, the HIC-HPLC pre-peak was lower, the SEC-HPLC monomer component was higher, the SEC-HPLC high-molecular-weight component was lower, and the methionine oxidation rate was lower. This suggests that the stability tends to increase as the content of the antioxidant methionine in the pH 5.5 formulation increases.

### (2) Comparison of Stability Depending on Antioxidant Content in pH 5.2 Formulation

From the results in Table 2 above, under the condition of temperature of 5±3°C, both Examples 2 and 4 had an HIC-HPLC main peak of 92% or more, an HIC-HPLC pre-peak of 4% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the conditions of temperature of 40±2°C and relative humidity of 75±5%, both Examples 2 and 4 had an HIC-HPLC main peak of 91% or more, an HIC-HPLC pre-peak of 5% or less, an HIC-HPLC post-peak of 5% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the condition of temperature of 45°C, both Examples 2 and 4 had an HIC-HPLC main peak of 91% or more, an HIC-HPLC pre-peak of 5% or less, an HIC-HPLC post-peak of 4% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the conditions of 12,000 lux light illumination, temperature of 25±2°C, relative humidity of 60±5%, and storage time of 72 hours, both Examples 2 and 4 had an HIC-HPLC main peak of 89% or more, an HIC-HPLC pre-peak of 7% or less, an HIC-HPLC post-peak of 4% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 10% or less, indicating that they were pharmaceutically acceptable stable examples.

In addition, it could be seen that, under the conditions of stirring at 1,000 RPM and storage time of 5 hours, both Examples 2 and 4 had an HIC-HPLC main peak of 92% or more, an HIC-HPLC pre-peak of 5% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, a heavy-chain Met105 oxidation rate of 1% or less, and a heavy-chain Met252 oxidation rate of 4% or less, indicating that they were pharmaceutically acceptable stable examples.

Through comparison of Examples 2 and 4 with Comparative Example 2, it could be confirmed that, as the content of methionine increased, the HIC-HPLC main peak was higher, the HIC-HPLC pre-peak was lower, the SEC-HPLC monomer component was higher, the SEC-HPLC high-molecular-weight component was lower, and the methionine oxidation rate was lower. This suggests that the stability tends to increase as the content of the antioxidant methionine in the pH 5.2 formulation increases.

### (3) Comparison of Stability Depending on pH of Formulation

From the results in Table 2 above, through comparison of Examples 1 to 4 and Comparative Examples 1 and 2, it was confirmed that the pH 5.2 formulation had a higher SEC-HPLC monomer component and a lower SEC-HPLC high-molecular-weight component than the pH 5.5 formulation, suggesting that the pH 5.2 formulation exhibits the best stability.

### (4) Comparison of Stability Depending on Type of Stabilizer

From the results in Table 2 above, it could be seen that, under the condition of temperature of 5±3°C, both Examples 2 and 5 had an HIC-HPLC main peak of 92% or more, an HIC-HPLC pre-peak of 4% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the conditions of temperature of 40±2°C and relative humidity of 75±5%, both Examples 2 and 5 had an HIC-HPLC main peak of 91% or more, an HIC-HPLC pre-peak of 5% or less, an HIC-HPLC post-peak of 5% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the condition of temperature of 45°C, both Examples 2 and 5 had an HIC-HPLC main peak of 91% or more, an HIC-HPLC pre-peak of 5% or less, an HIC-HPLC post-peak of 4% or less, an SEC-HPLC monomer component of 98% or more, an SEC-HPLC high-molecular-weight-component of 2% or less, an SEC-HPLC low-molecular-weight component of 1% or less, a heavy-chain Met105 oxidation rate of 2% or less, and a heavy-chain Met252 oxidation rate of 5% or less, indicating that they were pharmaceutically acceptable stable examples.

In addition, it could be seen that, under the conditions of 12,000 lux light illumination, temperature of 25±2°C, relative humidity of 60±5%, and storage time of 72 hours, both Examples 2 and 5 had an HIC-HPLC main peak of 89% or more, an HIC-HPLC pre-peak of 7% or less, an HIC-HPLC post-peak of 4% or less, an SEC-HPLC monomer component of 98% or more, an SEC-HPLC high-molecular-weight component of 2% or less, an SEC-HPLC low-molecular-weight component of 1% or less, a heavy-chain Met105 oxidation rate of 2% or less, and a heavy-chain Met252 oxidation rate of 10% or less, indicating that they were pharmaceutically acceptable stable examples.

In addition, it could be seen that, under the conditions of stirring at 1,000 RPM and storage time of 5 hours, both Examples 2 and 5 had an HIC-HPLC main peak of 92% or more, an HIC-HPLC pre-peak of 5% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, a heavy-chain Met105 oxidation rate of 1% or less, and a heavy-chain Met252 oxidation rate of 4% or less, indicating that they were pharmaceutically acceptable stable examples.

Through comparison of Examples 2 and 5 with Comparative Example 3, it was confirmed that, when sorbitol or proline was used as the stabilizer, the HIC-HPLC main-peak was higher, the HIC-HPLC pre-peak was lower, the SEC-HPLC monomer component was higher, the SEC-HPLC high-molecular-weight component was lower, and the methionine oxidation rate was lower than when sucrose was used, suggesting that the use of sorbitol or proline as a stabilizer exhibits better stability.

### (5) Long-Term Stability

From the results in Table 3 above, it could be seen that, under the condition of temperature of 5±3°C, both Examples 1-1 and 1-2 had an HIC-HPLC main peak of 92% or more, an HIC-HPLC pre-peak of 5% or less, an HIC-HPLC post-peak of 4% or less, an SEC-HPLC monomer component of 99% or more, an SEC-HPLC high-molecular-weight component of 1% or less, an SEC-HPLC low-molecular-weight component of 1% or less, an LC/MS heavy-chain Met105 oxidation rate of 2% or less, and a heavy-chain Met252 oxidation rate of 5% or less, indicating that they were pharmaceutically acceptable examples that are stable even when stored for a very long period of time.

## Claims

1. A stable pharmaceutical formulation comprising:
(A) an antibody or antigen-binding fragment thereof that binds to PD-1 (CD279, programmed cell death protein 1);
(B) a surfactant;
(C) a stabilizer;
(D) a buffer; and
(E) an antioxidant.

2. The stable pharmaceutical formulation of claim 1, wherein the antibody or antigen-binding fragment thereof (A) comprises: a light-chain variable region comprising a CDR1 domain comprising the amino acid sequence of SEQ ID NO:1, a CDR2 domain comprising the amino acid sequence of SEQ ID NO: 2, and a CDR3 domain comprising the amino acid sequence of SEQ ID NO: 3; and a heavy-chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

3. The stable pharmaceutical formulation of claim 1, wherein the antibody or antigen-binding fragment thereof (A) comprises a light-chain variable region comprising the amino acid sequence of SEQ ID NO: 7, and a heavy-chain variable region comprising the amino acid sequence of SEQ ID NO: 8.

4. The stable pharmaceutical formulation of claim 1, wherein the antibody or antigen-binding fragment thereof (A) comprises a light chain comprising the amino acid sequence of SEQ ID NO: 9, and a heavy chain comprising the amino acid sequence of SEQ ID NO: 10.

5. The stable pharmaceutical formulation of claim 1, wherein the antibody or antigen-binding fragment thereof (A) is pembrolizumab.

6. The stable pharmaceutical formulation of claim 1, wherein the pharmaceutical formulation is in a liquid state.

7. The stable pharmaceutical formulation of claim 1, wherein the pharmaceutical formulation is used for treating diseases associated with PD-1 (CD279, Programmed cell death protein 1).

8. The stable pharmaceutical formulation of claim 1, wherein a concentration of the antibody or antigen-binding fragment thereof (A) is 1 to 300 mg/ml.

9. The stable pharmaceutical formulation of claim 1, wherein the surfactant (B) comprises polysorbate, poloxamer, or a mixture thereof.

10. The stable pharmaceutical formulation of claim 9, wherein the polysorbate comprises at least one selected from the group consisting of polysorbate 20, polysorbate 40, polysorbate 60, and polysorbate 80.

11. The stable pharmaceutical formulation of claim 9, wherein the polysorbate comprises polysorbate 80.

12. The stable pharmaceutical formulation of claim 1, wherein a concentration of the surfactant (B) is 0.001 to 1% (w/v).

13. The stable pharmaceutical formulation of claim 1, wherein the stabilizer (C) comprises at least one selected from the group consisting of an amino acid or amino acid derivative, and a sugar or sugar alcohol.

14. The stable pharmaceutical formulation of claim 13, wherein the amino acid or amino acid derivative comprises at least one selected from the group consisting of threonine, arginine, proline, leucine, glycine, phenylalanine, tryptophan, glutamine, aspartate, glutamate, alanine, asparagine, serine, and tyrosine.

15. The stable pharmaceutical formulation of claim 13, wherein the sugar or sugar alcohol comprises at least one selected from the group consisting of trehalose, mannitol, sorbitol, glucose, fructose, galactose, xylose, maltose, lactose, xylitol, D-maltitol, inositol, lactitol, and isomalt.

16. The stable pharmaceutical formulation of claim 13, wherein a concentration of the amino acid or amino acid derivative is 10 to 450 mM.

17. The stable pharmaceutical formulation of claim 13, wherein a concentration of the sugar or sugar alcohol is 0.1 to 30% (w/v).

18. The stable pharmaceutical formulation of claim 1, wherein the stabilizer (C) comprises sorbitol.

19. The stable pharmaceutical formulation of claim 1, wherein the buffer (D) comprises at least one selected from the group consisting of acetic acid or a salt thereof, phosphoric acid or a salt thereof, citric acid or a salt thereof, succinic acid or a salt thereof, glutamic acid or a salt thereof, 2-(N-morpholino)ethanesulfonic acid (MES), and tromethamine (Tris) or a salt thereof.

20. The stable pharmaceutical formulation of claim 1, wherein a concentration of the buffer (D) is 0.1 to 50 mM.

21. The stable pharmaceutical formulation of claim 1, wherein the buffer (D) comprises acetic acid or a salt thereof.

22. The stable pharmaceutical formulation of claim 1, wherein the antioxidant (E) comprises at least one selected from the group consisting of methionine, ascorbic acid or a salt thereof, and taurine.

23. The stable pharmaceutical formulation of claim 1, wherein the antioxidant (E) comprises methionine.

24. The stable pharmaceutical formulation of claim 1, wherein a concentration of the antioxidant (E) is 0.1 to 100 mM.

25. The stable pharmaceutical formulation of claim 1, wherein the pharmaceutical formulation has a pH of 4 to 7.

26. The stable pharmaceutical formulation of claim 1, wherein the pharmaceutical formulation does not comprise sucrose.

27. The stable pharmaceutical formulation of claim 1, wherein the pharmaceutical formulation does not comprise histidine.

28. The stable pharmaceutical formulation of claim 1, wherein the pharmaceutical formulation is for intravenous administration or subcutaneous administration.

29. A stable pharmaceutical formulation comprising:
(A) 1 to 300 mg/ml of an antibody or antigen-binding fragment thereof comprising: a light-chain variable region comprising a CDR1 domain comprising the amino acid sequence of SEQ ID NO:1, a CDR2 domain comprising the amino acid sequence of SEQ ID NO: 2, and a CDR3 domain comprising the amino acid sequence of SEQ ID NO: 3; and a heavy-chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO:6;
(B) 0.001 to 1% (w/v) of a surfactant;
(C) 0.1 to 30% (w/v) of a sugar or sugar alcohol;
(D) 0.1 to 50 mM of a buffer; and
(E) 0.1 to 100 mM of an antioxidant.

30. The stable pharmaceutical formulation of claim 29, wherein the sugar or sugar alcohol (C) comprises sorbitol, the buffer (D) comprises acetic acid or a salt thereof, and the antioxidant (E) comprises methionine.

31. A vial filled with the stable pharmaceutical formulation set forth in any one of claims 1 to 30.

32. A cartridge filled with the stable pharmaceutical formulation set forth in any one of claims 1 to 30.

33. A pre-filled syringe filled with the stable pharmaceutical formulation set forth in any one of claims 1 to 30.

34. An auto-injector comprising therein the pre-filled syringe set forth in claim 33.
